# EUROPEAN PATENT SPECIFICATION

(11) **EP 3 941 448 B1**
(45) Date of publication and mention of the grant of the patent: **19.08.2026**
(21) Application number: 20711178.2
(22) Date of filing: 18.03.2020
(51) Int. Cl.: A61K 31/519, A61K 31/11, A61K 31/60, A61P 3/04, A61P 3/10, A61P 5/50

(54) **AGENTS CAPABLE OF INHIBITING THE ACTIVATION OF MAIT CELLS FOR USE IN THE TREATMENT OF OBESITY AND OBESITY-RELATED DISORDERS**
WIRKSTOFFE, DIE DIE AKTIVIERUNG VON MAIT-ZELLEN HEMMEN KÖNNEN, ZUR VERWENDUNG BEI DER BEHANDLUNG VON ADIPOSITAS UND ADIPOSITASBEDINGTEN ERKRANKUNGEN
AGENTS CAPABLES D'INHIBER L'ACTIVATION DES CELLULES MAIT À UTILISER DANS LE TRAITEMENT DE L'OBÉSITÉ ET DES TROUBLES LIÉS À L'OBÉSITÉ

(30) Priority: 19.03.2019 EP 19305332
(43) Date of publication of application: 26.01.2022
(73) Proprietor: Institut National de la Santé et de la Recherche Médicale (INSERM), 75013 Paris (FR); CENTRE NATIONAL DE LA RECHERCHE SCIENTIFIQUE (CNRS), 75016 Paris (FR); Université Paris Cité, 75006 Paris (FR)
(72) Inventor: LEHUEN-MONTEIRO, Agnès, 75014 Paris (FR); TOUBAL, Amine, 75014 PARIS (FR)
(74) Representative: Inserm Transfert
(86) International application number: PCT/EP2020/057368
(87) International publication number: WO 2020/187955

(56) References cited:
- WO-A1-2009/111889
- WO-A1-2018/234843
- MAGALHAES ET AL: "Mucosal-associated invariant T cell alterations in obese and type 2 diabetic patients.", J. CLIN. INVERT., vol. 125, no. 4, 9 March 2015 (2015-03-09) - 9 March 2015 (2015-03-09), pages 1752 - 1762, XP002794333
- APOSTOLOPOULOS VASSO; DE COURTEN MAXIMILIAN P J; STOJANOVSKA LILY; BLATCH GREGORY L; TANGALAKIS KATHY; DE COURTEN BARBORA: "he complex immunological and inflammatory network of adipose tissue in obesity.", MOL. NUTR. FOOD RES., vol. 60, 22 September 2015 (2015-09-22) - 22 September 2015 (2015-09-22), pages 43 - 57, XP002794334

## Description

### FIELD:

The present disclosure relates to the use of an agent capable of inhibiting the activation of MAIT cells for the treatment of obesity and obesity-related disorders.

### BACKGROUND:

Obesity involves low-grade inflammation in adipose tissue, with dysfunctional fat cells and increased immune cell infiltration. This chronic immune activity contributes to obesity-related conditions like type 2 diabetes, underscoring the need to understand immune-adipocyte interactions for better therapies (Apostolopoulos, Vasso, et al. "The complex immunological and inflammatory network of adipose tissue in obesity. "Molecular nutrition & food research 60.1 (2016): 43-57*).* In this context, WO2009111889 discloses that pharmaceutical approaches that modulate T may help treat, prevent, or reverse metabolic syndrome and type 2 diabetes by altering the immune environment in adipose tissue and improving insulin sensitivity.

Mucosal-associated invariant T (MAIT) cells are a subset of innate-like immune cells found in peripheral blood, intestinal mucosa, and abundantly in human liver. MAIT cells express an invariant T cell receptor α chain, the Vα7.2-Jα33 chain in humans. MAIT cells can produce IFN-γ, granzyme B (GrB), and IL-17; are restricted by the major histocompatibility complex class I-related molecule MR1; and are activated by cells infected by different microorganisms. Vitamin B2 (riboflavin) metabolites produced by bacteria and yeasts are required to generate MAIT cell-activating ligands. WO2018234843 discloses that MAIT cells contribute to fibrosis progression, and inhibiting their activation may offer a promising therapeutic strategy for treating chronic liver injury. It has also been recently shown that circulating MAIT cell frequency was dramatically decreased in the blood and adipose tissues of patients with T2D and/or severe obesity (Magalhaes, Isabelle, et al. "Mucosal-associated invariant T cell alterations in obese and type 2 diabetic patients." The Journal of clinical investigation 125.4 (2015): 1752-1762*).* This study also revealed that this population was even undetectable in some obese patients. Moreover, in both patient groups, circulating MAIT cells displayed an activated phenotype that was associated with elevated Th1 and Th17 cytokine production. In obese patients, MAIT cells were more abundant in adipose tissue than in the blood and exhibited a striking IL-17 profile. The same study showed that bariatric surgery in obese patients not only improved their metabolic parameters but also increased circulating MAIT cell frequency at 3 months after surgery. This study thus reveals profound MAIT cell abnormalities in patients harboring metabolic disorders, suggesting their potential role in these pathologies.

### SUMMARY OF THE INVENTION:

The present invention is defined by the claims. In particular, the present invention relates to:
- an agent capable of inhibiting the activation of MAIT cells for use in a method of treating obesity in a subject in need thereof wherein the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA),
- an agent capable of inhibiting the activation of MAIT cells for use in a method of treating insulin resistance in a subject in need thereof wherein the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA),
- an agent capable of inhibiting the activation of MAIT cells for use in a method of treating type 2 diabetes in a subject in need thereof wherein the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA) and,
- an agent capable of inhibiting the activation of MAIT cells for use in a method of treating metabolic syndrome in a subject in need thereof wherein the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA).

The following detailed description, figures and example are present for understanding and illustration purposes only.

Furthermore, any reference in the description to methods of treatment refer to the compounds, pharmaceutical compositions and medicaments of the present invention for use in a method of treatment of the human body by therapy.

### DETAILED DESCRIPTION:

Obesity is associated with low-grade inflammation in adipose tissue (AT) and dysfunctional adipocytes producing inflammatory molecules. A recent study reveals profound MAIT cell abnormalities in patients harboring metabolic disorders, suggesting their potential role in these pathologies (Magalhaes, Isabelle, et al. "Mucosal-associated invariant T cell alterations in obese and type 2 diabetic patients. " The Journal of clinical investigation 125.4 (2015): 1752-1762*).* Now the inventors show that MAIT cells induce adipose tissue and ileum dysfunction and inflammation in obese mice. Moreover, the inventors show that a treatment with an agent capable of inhibiting the activation of MAIT cells (i.e. Ac-6-FP) during high fat diet (HFD) improved metabolic parameters and in particular insulin sensitivity. Thus the present disclosure relates to the use of an agent capable of inhibiting the activation of MAIT cells for the treatment of obesity and obesity-related disorders.

In particular, an object of the present disclosure relates to a method of treating obesity in a subject in need thereof comprising administering to the subject a therapeutically effective amount of an agent capable of inhibiting the activation of MAIT cells.

A further object of the present disclosure relates to a method of treating insulin resistance in a subject in need thereof (e.g. an obese subject) comprising administering to the subject a therapeutically effective amount of an agent capable of inhibiting the activation of MAIT cells.

A further object of the present disclosure relates to a method of treating type 2 diabetes in a subject in need thereof (e.g. an obese subject) comprising administering to the subject a therapeutically effective amount of an agent capable of inhibiting the activation of MAIT cells.

A further object of the present disclosure relates to a method of metabolic syndrome in a subject in need thereof (e.g. an obese subject) comprising administering to the subject a therapeutically effective amount of an agent capable of inhibiting the activation of MAIT cells.

As used herein, the term "subject" refers to a human or another mammal (e.g., primate, dog, cat, goat, horse, pig, mouse, rat, rabbit, and the like), that can be afflicted with obesity. In particular, the subject is a human being. More particularly, the subject is often referred to as an "individual". The term "individual" does not denote a particular age, and thus encompasses children, teenagers, and adults.

As used herein the term "obesity" refers to a condition characterized by an excess of body fat. The operational definition of obesity is based on the Body Mass Index (BMI), which is calculated as body weight per height in meter squared (kg/m²). Obesity refers to a condition whereby an otherwise healthy subject has a BMI greater than or equal to 30 kg/m², or a condition whereby a subject with at least one co-morbidity has a BMI greater than or equal to 27 kg/m². An "obese subject" is an otherwise healthy subject with a BMI greater than or equal to 30 kg/m² or a subject with at least one co-morbidity with a BMI greater than or equal 27 kg/m². A "subject at risk of obesity" is an otherwise healthy subject with a BMI of 25 kg/m² to less than 30 kg/m² or a subject with at least one co-morbidity with a BMI of 25 kg/m² to less than 27 kg/m². The increased risks associated with obesity may occur at a lower BMI in people of Asian descent. In Asian and Asian-Pacific countries, including Japan, "obesity" refers to a condition whereby a subject with at least one obesity-induced or obesity-related co-morbidity that requires weight reduction or that would be improved by weight reduction, has a BMI greater than or equal to 25 kg/m². An "obese subject" in these countries refers to a subject with at least one obesity-induced or obesity-related co-morbidity that requires weight reduction or that would be improved by weight reduction, with a BMI greater than or equal to 25 kg/m². In these countries, a "subject at risk of obesity" is a person with a BMI of greater than 23 kg/m² to less than 25 kg/m².

As used herein, the term "insulin resistance" has its common meaning in the art. Insulin resistance is a physiological condition where the natural hormone insulin becomes less effective at lowering blood sugars. The resulting increase in blood glucose may raise levels outside the normal range and cause adverse health effects such as metabolic syndrome, dyslipidemia and subsequently type 2 diabetes mellitus.

As used herein, the term "type 2 diabetes" or "non-insulin dependent diabetes mellitus (NIDDM)" has its general meaning in the art. Type 2 diabetes often occurs when levels of insulin are normal or even elevated and appears to result from the inability of tissues to respond appropriately to insulin. Most of the type 2 diabetics are obese.

As used herein, the term "Metabolic Syndrome" refers to a subject characterized by having three or more of the following symptoms: abdominal obesity, hyperglyceridemia, low HDL cholesterol, high blood pressure, and high fasting plasma glucose. The criteria for these symptoms are defined in the third Report of the National Cholesterol Education Program Expert Panel in Detection, Evaluation and Treatment of High blood Cholesterol in Adults (Ford, E S. et al. 2002).

As used herein, the term "treatment" or "treat" refer to both prophylactic or preventive treatment as well as curative or disease modifying treatment, including treatment of subject at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]). In particular, the method herein disclosed is particularly suitable for improving blood glucose control, enhancing insulin signalling in skeletal muscle and adipose tissue, reducing lipotoxicity in skeletal muscle and adipose tissue, increasing lipid oxidative capacity in skeletal muscle and adipose tissue, or maintaining long-term insulin sensitivity in the subject.

As used herein, the term "MAIT cells" or "Mucosal-Associated Invariant T cells" refers to a population of T cells present in mammals, preferably humans, that display an invariant TCR alpha chain comprising Vα7.2-Jα33 (in humans), a CDR3 of constant length, and a limited number of Vβ segments together with an activated phenotype (CD44) (see, e.g., Lantz and Bendelac. 1994. J. Exp Med. 180:1097-106; Tilloy et al., J. Exp. Med., 1999, 1907-1921; Treiner et al. (2003) Nature 422:164-169). MAIT cells are generally CD8⁺ (expressing mostly the homodimeric form of CD8αα) or CD4⁻/CD8⁻ (DN), and are restricted by the non-classical MHC class I molecule MR1. For the purposes of the present disclosure, any T cells that express the invariant Vα7.2-Jα33 alpha TCR chain are considered to be MAIT cells. Typically, the alpha chain is associated with an invariant CDR3 and with either Vβ2 or Vβ13.

As used herein, the expression "agent capable of inhibiting the activation of MAIT cells" refers to any refers to any molecule that under cellular and/or physiological conditions is capable of inhibiting the pro-inflammatory functions of MAIT cells.

In particular, the agent is a small organic molecule. Inhibitors of MAIT cells are known in the art and typically include those described in Corbett, A.J. et al. T-cell activation by transitory neo-antigens derived from distinct microbial pathways. Nature 509, 361-365 (2014); and Keller AN et al. Drugs and drug-like molecules can modulate the function of mucosal-associated invariant T cells Nat Immunol. 2017 Apr;18(4):402-411. Other examples include those described in the International Patent Application WO 2014005194. In particular, the inhibitor is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA).

A "therapeutically effective amount" refers to an amount effective of the agent, at dosages and for periods of time necessary, to achieve a desired therapeutic result. A therapeutically effective amount of drug may vary according to factors such as the disease state, age, sex, and weight of the individual, and the ability of drug to elicit a desired response in the individual. A therapeutically effective amount is also one in which any toxic or detrimental effects of the antibody or antibody portion are outweighed by the therapeutically beneficial effects. The efficient dosages and dosage regimens for drug depend on the disease or condition to be treated and may be determined by the persons skilled in the art. A physician having ordinary skill in the art may readily determine and prescribe the effective amount of the pharmaceutical composition required. For example, the physician could start doses of drug employed in the pharmaceutical composition at levels lower than that required in order to achieve the desired therapeutic effect and gradually increase the dosage until the desired effect is achieved. In general, a suitable dose of a composition of the present disclosure will be that amount of the compound which is the lowest dose effective to produce a therapeutic effect according to a particular dosage regimen. Such an effective dose will generally depend upon the factors described above.

Typically, the agent herein disclosed is administered to the subject in the form of a pharmaceutical composition which comprises a pharmaceutically acceptable carrier. Pharmaceutically acceptable carriers that may be used in these compositions include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a subject, the composition will be formulated for administration to the subject. The compositions herein disclosed may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions herein disclosed may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions herein disclosed may be orally administered in any orally acceptable dosage form including capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions herein disclosed may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions herein disclosed may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds herein disclosed include mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions herein disclosed may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. For example, an antibody present in a pharmaceutical composition herein disclosed can be supplied at a concentration of 10 mg/mL in either 100 mg (10 mL) or 500 mg (50 mL) single-use vials. The product is formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. An exemplary suitable dosage range for an antibody in a pharmaceutical composition herein disclosed may between about 1 mg/m² and 500 mg/m². However, it will be appreciated that these schedules are exemplary and that an optimal schedule and regimen can be adapted taking into account the affinity and tolerability of the particular antibody in the pharmaceutical composition that must be determined in clinical trials. A pharmaceutical composition herein disclosed for injection (e.g., intramuscular, i.v.) could be prepared to contain sterile buffered water (e.g. 1 ml for intramuscular), and between about 1 ng to about 100 mg, e.g. about 50 ng to about 30 mg or more preferably, about 5 mg to about 25 mg, of agent herein disclosed.

The present disclosure will be further illustrated by the following figures and examples. However, these examples and figures should not be interpreted in any way as limiting the scope of the present disclosure.

### FIGURES:

Figure 1: ITT and OGTT in Vα19^{+/-} and MR1^{-/-} and their littermate controls (CTL) fed with HFD during 12 weeks (n=5-6 mice/group).
Figure 2: Ac-6-FP treatment during HFD improved metabolic parameters. Vα19^{+/-} mice fed during 6 weeks of HFD were then giving water containing 50nM/ml of Ac-6-FP (Acetyl-6-formylpterin) (n=8) or PBS (n=7) for 8 weeks and IP injected, twice a week, 50nM of Ac-6-FP or PBS. (A) ITT and OGTT in Vα19^{+/-} Ac-6-FP-treated mice and Vα19^{+/-} control mice. (B) Percentage of weight gain of Ac-6-FP-treated Vα19^{+/-} mice and Vα19^{+/-} control mice.
Figure 3: Intra cellular staining of MAIT cells for IL-17A. Frequency of IL-17A positive MAIT cells in ileum and visceral adipose tissue from Vα19+/- control (PBS) and Vα19+/- Ac-6-FP treated mice.
Figure 4: Frequency of M1-macrophages (CD206-CD11c+) and M2-macrophages (CD206+CD11c-) among total macrophages and M1/M2 ratio in visceral adipose tissue from Vα19+/- Ac-6-FP treated mice and their controls.
Figure 5: Insulin Tolerance Test (ITT) and Oral Glucose Tolerance Test (OGTT) at 12 weeks of High Fat Diet (HFD) in MR1-/- Ac-6-FP treated mice and their respective controls.

### EXAMPLE 1:

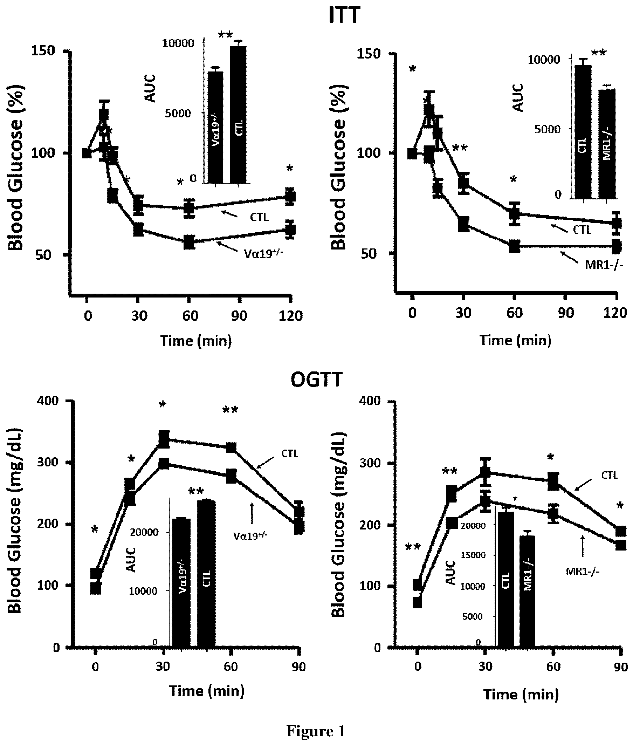

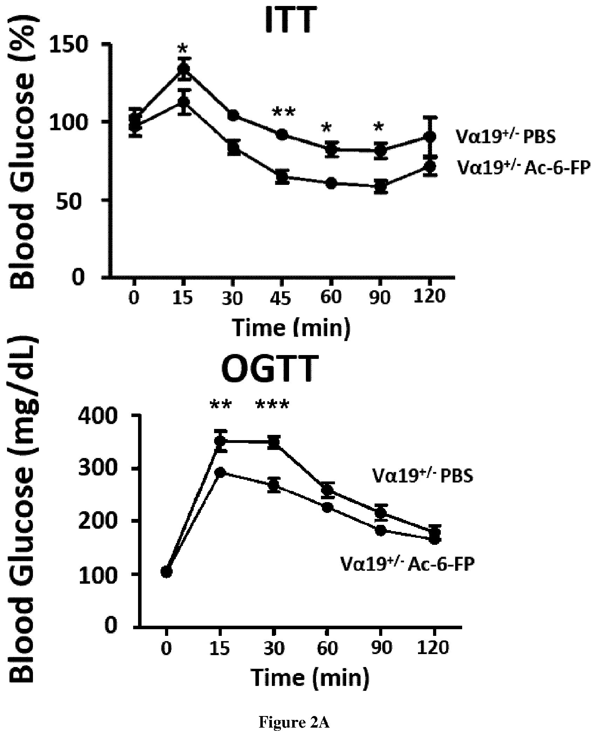

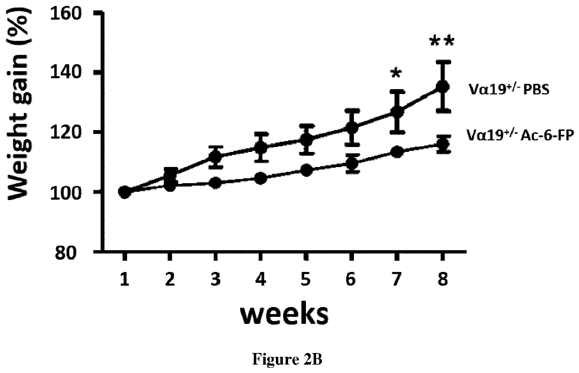

Obesity is associated with low-grade inflammation in adipose tissue (AT) and dysfunctional adipocytes producing inflammatory molecules. A recent study reveals profound MAIT cell abnormalities in patients harboring metabolic disorders, suggesting their potential role in these pathologies *(*Magalhaes, Isabelle, et al. "Mucosal-associated invariant T cell alterations in obese and type 2 diabetic patients. " The Journal of clinical investigation 125.4 (2015): 1752-1762*).* Now the inventors show that MAIT cells induce adipose tissue and ileum dysfunction and inflammation in obese mice. Moreover, the inventors show that a treatment with an agent capable of inhibiting the activation of MAIT cells (i.e. Ac-6-FP) during high fat diet (HFD) improved metabolic parameters and in particular insulin sensitivity. In particular, the results are depicted in **Figures 1** **and** **2A****-2B.** Thus the present disclosure relates to the use of an agent capable of inhibiting the activation of MAIT cells for the treatment of obesity and obesity-related disorders.

### EXAMPLE 2:

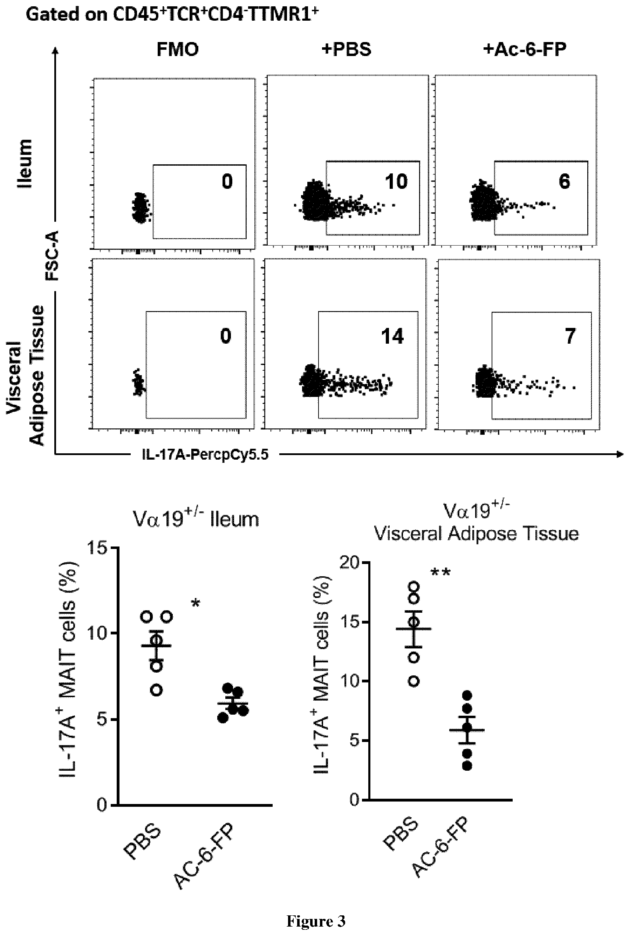

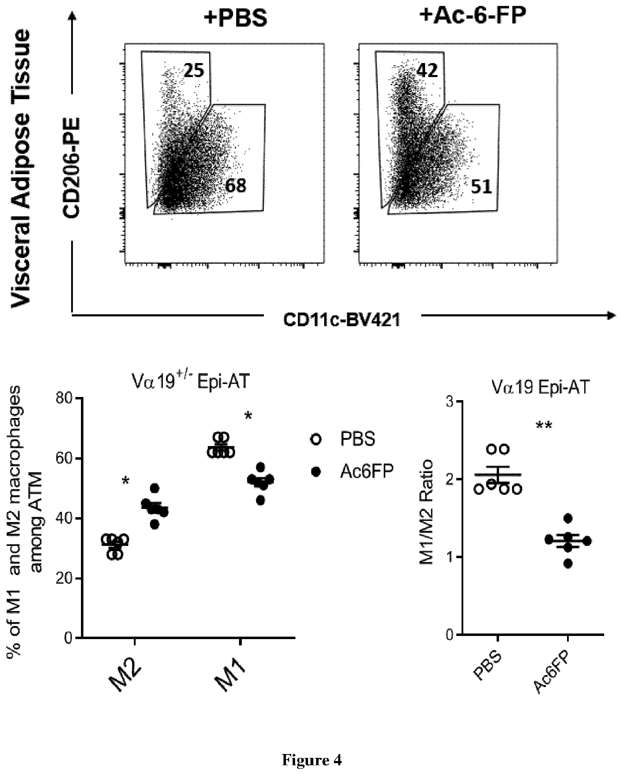

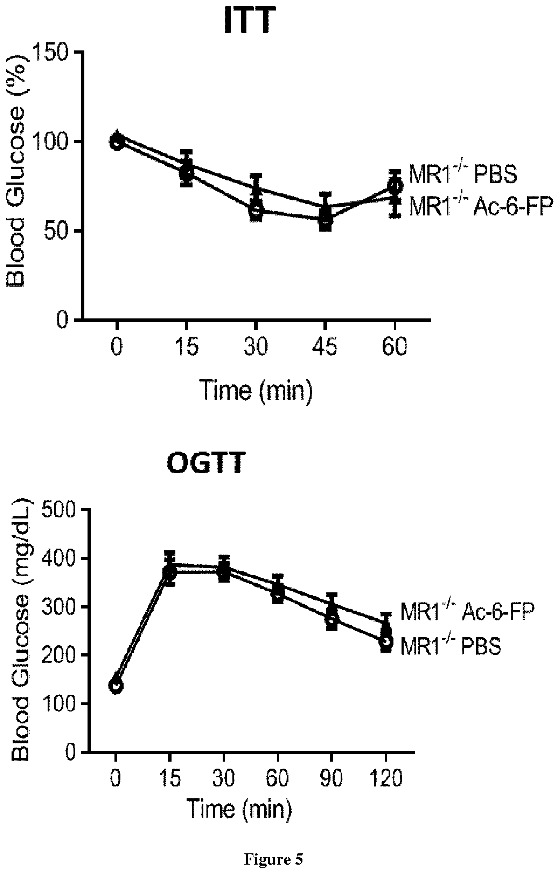

Blocking MAIT cells activation with Ac-6-FP treatment of Vα19+/- mice induced a decreased production of IL-17A by MAIT cells in both ileum and visceral adipose tissue (Figure 3). Interestingly Ac-6-FP treatment also impacted visceral adipose tissue macrophages polarization of Vα19+/- mice. Treated mice harboured an increased frequency of M2-like macrophages (anti-inflammatory), and a decreased frequency of M1-like macrophages (pro-inflammatory) when compared to non-treated mice (Figure 4). Finally, Ac-6-FP treatment of MR1-/- mice did not have any effect on their glucose tolerance or insulin sensitivity, strengthening the key role of MR1-TCR interaction in inflammation and metabolism dysregulation induced by MAIT cells (Figure 5).

### REFERENCES:

Throughout this application, various references describe the state of the art to which this disclosure pertains.

## Claims

1. An agent capable of inhibiting the activation of MAIT cells for use in a method of treating obesity in a subject in need thereof wherein:
the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA).

2. An agent capable of inhibiting the activation of MAIT cells for use in a method of treating insulin resistance in a subject in need thereof wherein:
- the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA).

3. An agent capable of inhibiting the activation of MAIT cells for use in a method of treating type 2 diabetes in a subject in need thereof wherein:
- the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA).

4. An agent capable of inhibiting the activation of MAIT cells for use in a method of treating metabolic syndrome in a subject in need thereof wherein:
- the agent is selected from the group consisting of 6-formyl pterin, acetyl-6-formylpterin (Ac-6-FP), 3-formylsalicylic acid (3-F-SA), 5-formylsalicylic acid (5-F-SA) and 2-hydroxy-1-naphthaldehyde (2-OH-1-NA).

## Patentansprüche

1. Wirkstoff, der die Aktivierung von MAIT-Zellen hemmen kann, zur Verwendung in einem Verfahren zur Behandlung von Fettleibigkeit bei einem behandlungsbedürftigen Patienten, wobei:
der Wirkstoff aus der Gruppe bestehend aus 6-Formylpterin, Acetyl-6-formylpterin (Ac-6-FP), 3-Formylsalicylsäure (3-F-SA), 5-Formylsalicylsäure (5-F-SA) und 2-Hydroxy-1-naphthaldehyd (2-OH-1-NA) ausgewählt ist.

2. Wirkstoff, der die Aktivierung von MAIT-Zellen hemmen kann, zur Verwendung in einem Verfahren zur Behandlung von Insulinresistenz bei einem behandlungsbedürftigen Patienten, wobei:
- der Wirkstoff aus der Gruppe bestehend aus 6-Formylpterin, Acetyl-6-formylpterin (Ac-6-FP), 3-Formylsalicylsäure (3-F-SA), 5-Formylsalicylsäure (5-F-SA) und 2-Hydroxy-1-naphthaldehyd (2-OH-1-NA) ausgewählt ist.

3. Wirkstoff, der die Aktivierung von MAIT-Zellen hemmen kann, zur Verwendung in einem Verfahren zur Behandlung von Typ-2-Diabetes bei einem behandlungsbedürftigen Patienten, wobei:
- der Wirkstoff aus der Gruppe bestehend aus 6-Formylpterin, Acetyl-6-formylpterin (Ac-6-FP), 3-Formylsalicylsäure (3-F-SA), 5-Formylsalicylsäure (5-F-SA) und 2-Hydroxy-1-naphthaldehyd (2-OH-1-NA) ausgewählt ist.

4. Wirkstoff, der die Aktivierung von MAIT-Zellen hemmen kann, zur Verwendung in einem Verfahren zur Behandlung eines metabolischen Syndroms bei einem behandlungsbedürftigen Patienten, wobei:
- der Wirkstoff aus der Gruppe bestehend aus 6-Formylpterin, Acetyl-6-formylpterin (Ac-6-FP), 3-Formylsalicylsäure (3-F-SA), 5-Formylsalicylsäure (5-F-SA) und 2-Hydroxy-1-naphthaldehyd (2-OH-1-NA) ausgewählt ist.

## Revendications

1. Agent capable d'inhiber l'activation des cellules MAIT, destiné à être utilisé dans un procédé de traitement de l'obésité chez un sujet qui en a besoin, dans lequel :
- l'agent est sélectionné dans le groupe constitué de la 6-formylptérine, de l'acétyl-6-formylptérine (Ac-6-FP), de l'acide 3-formylsalicylique (3-F-SA), de l'acide 5-formylsalicylique (5-F-SA) et du 2-hydroxy-1-naphtaldéhyde (2-OH-1-NA).

2. Agent capable d'inhiber l'activation des cellules MAIT, destiné à être utilisé dans un procédé de traitement de la résistance à l'insuline chez un sujet qui en a besoin, dans lequel :
- l'agent est sélectionné dans le groupe constitué de la 6-formylptérine, de l'acétyl-6-formylptérine (Ac-6-FP), de l'acide 3-formylsalicylique (3-F-SA), de l'acide 5-formylsalicylique (5-F-SA) et du 2-hydroxy-1-naphtaldéhyde (2-OH-1-NA).

3. Agent capable d'inhiber l'activation des cellules MAIT, destiné à être utilisé dans un procédé de traitement du diabète de type 2 chez un sujet qui en a besoin, dans lequel :
- l'agent est sélectionné dans le groupe constitué de la 6-formylptérine, de l'acétyl-6-formylptérine (Ac-6-FP), de l'acide 3-formylsalicylique (3-F-SA), de l'acide 5-formylsalicylique (5-F-SA) et du 2-hydroxy-1-naphtaldéhyde (2-OH-1-NA).

4. Agent capable d'inhiber l'activation des cellules MAIT, destiné à être utilisé dans un procédé de traitement du syndrome métabolique chez un sujet qui en a besoin, dans lequel :
- l'agent est sélectionné dans le groupe constitué de la 6-formylptérine, de l'acétyl-6-formylptérine (Ac-6-FP), de l'acide 3-formylsalicylique (3-F-SA), de l'acide 5-formylsalicylique (5-F-SA) et du 2-hydroxy-1-naphtaldéhyde (2-OH-1-NA).
